# EUROPÄISCHE PATENTSCHRIFT

(11) **EP 2 509 014 B1**
(45) Veröffentlichungstag und Bekanntmachung des Hinweises auf die Patenterteilung: **02.01.2019**
(21) Anmeldenummer: 12001252.1
(22) Anmeldetag: 25.02.2012
(51) Int. Cl.: G06F 19/00

(54) **Elektronisches Installationsgerät zur Erfassung und Abspeicherung von Gesundheits-, Fitness-, Wellness- und Vital-Daten**
Electronic installation device for recording and storing health, fitness, wellness and vitality data
Appareil d'installation électronique destiné à la saisie et l'enregistrement de données de santé, de forme, de bien-être et de signes vitaux

(30) Priorität: 08.04.2011 DE 102011016546
(43) Veröffentlichungstag der Anmeldung: 10.10.2012
(73) Patentinhaber: ABB AG, 68309 Mannheim (DE)
(72) Erfinder: Lehnert, Christian, 58239 Schwerte (DE)

(56) Entgegenhaltungen:
- EP-A2- 2 302 922
- WO-A2-2010/072387
- US-B1- 6 406 426

## Beschreibung

Die Erfindung betrifft ein elektronisches Installationsgerät zur Erfassung und Abspeicherung von Gesundheits-, Fitness-, Wellness- und Vital-Daten, wobei sinnvolle Einsatzorte des elektronischen Installationsgerätes z. B. Bad oder Saunavorraum sind. Das vorgeschlagene elektronische Gerät kann in Form eines Unterputzgerätes oder Aufputzgerätes in einem Elektro-Installationssystem realisiert werden.

Zur Erfassung von Gesundheits-, Fitness-, Wellness- und Vital-Daten sind Einzelgeräte / Einzelsysteme für die separierte Sammlung von Informationen/Daten in Teilbereichen allgemein bekannt, welche unterschiedliche Systeme zur Auswertung und Darstellung von erfassten Daten nutzen, wie z. B. Waage, Pulsuhr und Software-Lösungen auf PC-Basis, wie z. B. USB-Blutzuckermessgeräte.

Der Erfindung liegt die Aufgabe zugrunde, ein optimiertes elektronisches Installationsgerät zur Erfassung und Abspeicherung von Gesundheits-, Fitness-, Wellness- und Vital-Daten anzugeben.

Diese Aufgabe wird erfindungsgemäß gelöst durch ein elektronisches Installationsgerät zur Erfassung und Abspeicherung von Gesundheits-, Fitness-, Wellness- und Vital-Daten,
- mit mindestens einer Erfassungs-Schnittstelle für die Erfassung von Daten mindestens eines externen Gesundheits-, Fitness-, Wellness- und Vital-Daten-Gerätes,
- mit einem Speicher zum Abspeichern dieser erfassten Daten,
- mit einer Steuerlogik/Auswertung/Verarbeitung zur Auswertung / Verarbeitung dieser erfassten Daten und zur Bereitstellung hierauf basierender relevanter Informationen,
- mit einem Netzteil und
- mit einer Personalisierungs-Schnittstelle zur Unterscheidung der Daten unterschiedlicher Geräte-Anwender.

Die mit der Erfindung erzielbaren Vorteile liegen insbesondere darin, dass eine zentrale Speicherung, Auswertung und Darstellung aller erfassten Gesundheits-, Fitness-, Wellness- und Vital-Daten in einem einzigen Gerät der elektrischen Installationstechnik ermöglicht wird. Darüber hinaus wird die Voraussetzung für eine Erstellung von datenübergreifenden / geräteübergreifenden Auswertungen und von Entwicklungen (Historien) dieser Gesundheits-, Fitness-, Wellness- und Vital-Daten über eine vorgebbare Zeitperiode geschaffen. Des Weiteren wird der Weg für eine direkte Interaktion mit der Gebäudesystemtechnik geebnet, z. B. bei Überschreitung vorgegebener Grenzwerte.

Sofern es die einzelnen Gesundheits-, Fitness-, Wellness- und Vital-Daten-Geräte nicht bereits bieten, können mittels der Steuerlogik/Auswertung/Verarbeitung abgeleitete Informationen aus den erfassten Grund-Daten im elektronischen Installationsgerät zur Erfassung und Abspeicherung von Gesundheits-, Fitness-, Wellness- und Vital-Daten berechnet und dargestellt werden. Das vorgeschlagene elektronische Installationsgerät zur Erfassung und Abspeicherung für Gesundheits-, Fitness-, Wellness- und Vitaldaten dient insgesamt einer einfachen und flexiblen, d.h. leicht anzupassenden Darstellung und Auswertung im Zusammenhang mit der permanenten Speicherung und Trendanalyse, mit der Möglichkeit der Interaktion der Gebäudesystemtechnik und deren Steuerung. Besondere Vorteile bestehen in der einfachen Handhabung, der Modularität bezogen auf die Erfassung von Daten verschiedener Personen und damit der universellen Nutzung durch den Endkunden.

Unter dem Begriff "Elektro-Installationssystem" wird das Gebiet der Installationstechnik für Wohn- und Zweckbau verstanden. Unter dem Begriff "Gebäudesystemtechnik" wird das Gebiet der Gebäudeautomation eines Elektro-Installationssystems verstanden.

Die Erfindung wird nachstehend an Hand der in der Zeichnung dargestellten Ausführungsbeispiele erläutert. Es zeigen:
- Fig. 1: eine schematische Übersicht mit einem elektronischen Installationsgerät zur Erfassung und Abspeicherung von Gesundheits-, Fitness-, Wellness- und Vital-Daten mit den zuordenbaren Gesundheits-, Fitness-, Wellness- und Vital-Daten-Geräten,
- Fig. 2: eine erste Ausführungsform eines elektronisches Installationsgeräts zur Erfassung und Abspeicherung von Gesundheits-, Fitness-, Wellness- und Vital-Daten,
- Fig. 3: eine zweite Ausführungsform eines elektronisches Installationsgeräts zur Erfassung und Abspeicherung von Gesundheits-, Fitness-, Wellness- und Vital-Daten,
- Fig. 4: eine dritte Ausführungsform eines elektronisches Installationsgeräts zur Erfassung und Abspeicherung von Gesundheits-, Fitness-, Wellness- und Vital-Daten,
- Fig. 5: eine vierte Ausführungsform eines elektronisches Installationsgeräts zur Erfassung und Abspeicherung von Gesundheits-, Fitness-, Wellness- und Vital-Daten.

Unter den Gesundheits-, Fitness-, Wellness- und Vital-Daten einer bestimmten Person werden im Nachfolgenden beispielhaft folgende Daten / Informationen verstanden:
- Gesundheits-Daten:
   a) Gewicht
   b) Körperfettanteil
   c) Körperwasseranteil
   d) Grundumsatz
   e) Stoffwechselalter
   f) Knochenmasse
   g) Muskelmasse
   h) Körperbauwert
   i) Blutzuckergehalt
- Fitnessdaten:
   j) Distanz
   k) Geschwindigkeit
   I) Trittfrequenz (Radfahren)
   m) Lauffrequenz (Laufen / Jogging)
- Vital-Daten
   n) Herzfrequenz
   o) Atemfrequenz
   p) Blutdruck
   q) Körpertemperatur
   r) Alphafrequenz (Elektroenzephalographie, EEG)
   s) Hautleitwert
   t) Pupillengröße
- Sonstige Daten inklusive Wellness-/Bio-Feedback-Daten:
   u) Gewicht von Speisen und/oder Zutaten
   v) Zusammensetzung von Speisen und/oder Zutaten (wie z. B. Fettanteil, Milchanteil,...)
   w) Umweltdaten (Temperatur, Luftfeuchte, CO2,...)
   x) Biorhythmus
   y) Organuhr (gemäß der Chinesischen Medizin)
   z) Mondphasen

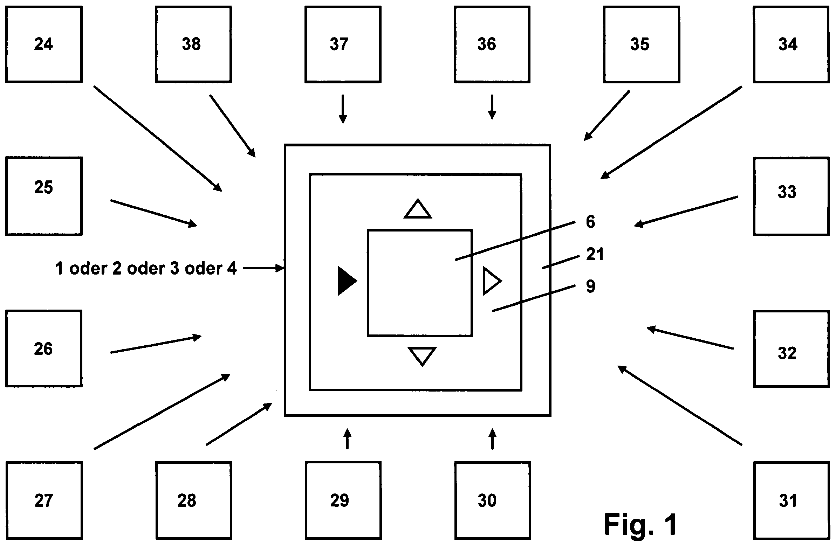

In Fig. 1 ist eine schematische Übersicht mit einem elektronischen Installationsgerät zur Erfassung und Abspeicherung von Gesundheits-, Fitness-, Wellness- und Vital-Daten mit den zugeordneten Gesundheits-, Fitness-, Wellness- und Vital-Daten-Geräten dargestellt. Prinzipiell können dabei die Daten der folgenden Gesundheits-, Fitness-, Wellness- und Vital-Daten-Geräte 24 - 38 an ein elektronisches Installationsgerät 1 oder 2 oder 3 oder 4 zur Erfassung und Abspeicherung von Gesundheits-, Fitness-, Wellness- und Vital-Daten kabellos oder kabelgebunden weitergeleitet werden:
- die Daten eine Körper-Analyse-Waage 24 - siehe z. B. b), c), f), g),
- die Daten einer Personenwaage 25 - siehe z. B. a),
- die Daten einer Küchenwaage 26 - siehe z. B. u),
- die Daten eines Testgerätes 27 für Alkohol oder ähnliche Stoffe,
- die Daten eines Lauf-/Trittsensors 28 - siehe z. B. j), k), l), m),
- die Daten eines Kraft-/Leistungssensors 29 - siehe z. B. k), I), m),
- die Daten eines Pulssensors 30 - siehe z. B. n),
- die Daten eines Ergometers / Trimmrads 31 - siehe z. B. j), k), l), m),
- die Daten einer Wetterstation 32 - siehe z. B. w), z),
- die Daten mindestens eines Umweltmesssensors 33 - siehe z. B. w),
- die Daten eines Hautleitsensors 34 - siehe z. B. s),
- die Daten eines EEG-Gerätes 35 - siehe z. B. n), r),
- die Daten eines Bio-Feedback-Gerätes 36 - siehe z. B. n), o), p), q), t),
- die Daten eines Blutzuckermessgerätes 37 - siehe z. B. i),
- die Daten eines Raumluftsensors 38 - siehe z. B. w).

Das elektronische Installationsgerät 1 oder 2 oder 3 oder 4 zur Erfassung und Abspeicherung von Gesundheits-, Fitness-, Wellness- und Vital-Daten weist dabei beispielsweise eine zentrale Anzeigeeinheit 6, ein in Form mehrerer Taster ausgebildetes Bedienelement 9 - eine "schwimmende Wippe", welche wahlweise rechts / links / oben / unten betätigbar ist, um derart gewünschte Bedienfunktionen auszuüben - und einen Abdeckrahmen 21 auf.

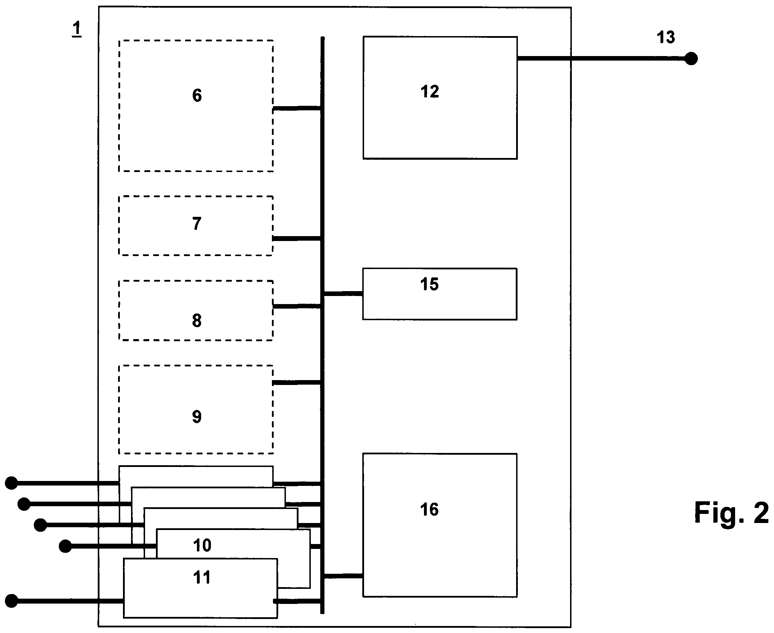

In Fig. 2 ist eine erste Ausführungsform eines elektronischen Installationsgeräts zur Erfassung und Abspeicherung von Gesundheits-, Fitness-, Wellness- und Vital-Daten dargestellt. Das elektronische Installationsgerät 1 umfasst:
- optional eine Anzeigeeinheit 6 zur Visualisierung von Daten (Text, Grafik) und/oder Signalisierung, z. B. Farbsignalisierung bei Überschreitung von Grenzwerten,
- optional einen Lautsprecher 7,
- optional ein Mikrofon 8
- optional ein (bzw. mehrere) Bedienelement(e) 9, z. B. in Form mindestens eines Tasters oder in Form eines Touch Screens,
- mindestens eine (vorzugsweise mehrere) Erfassungs-Schnittstelle(n) 10 zu externen Gesundheits-, Fitness-, Wellness- und Vital-Daten-Geräten, z. B. externen Sensoren / Erfassungsgeräten, wobei diese Erfassungs-Schnittstelle(n) 10 kabellos oder kabelgebunden ausgebildet sein kann (können),
- eine Personalisierungs-Schnittstelle 11
- ein Netzteil 12, welches an eine externe Spannungsversorgung 13 z. B. an ein 230V-Wechsselspannungsnetz angeschlossen ist,
- eine Steuerlogik/Auswertung/Verarbeitung 15 und
- einen Speicher 16 zum Abspeichern von erfassten / berechneten / ausgewerteten / verarbeiteten Daten und gegebenenfalls vorgegebenen Schwellwerten.

Die Baueinheiten 6, 7, 8, 9, 10, 11, 15, 16 (soweit vorhanden) sind über Datenleitungen miteinander verbunden. Das Netzteil 12 versorgt alle Baueinheiten des elektronischen Installationsgeräts 1.

Die Anzeigeeinheit 6 kann aus LEDs aufgebaut sein oder in Form einer flächigen Anzeige aus LCD mit/ohne LED-Hinterleuchtung oder auf Basis einer OLED-Anzeige, die entweder eine LCD-Anzeige farbig hinterleuchtet oder die Information in Farbe und Text (Text- / Grafikdisplay) anzeigt, bestehen.

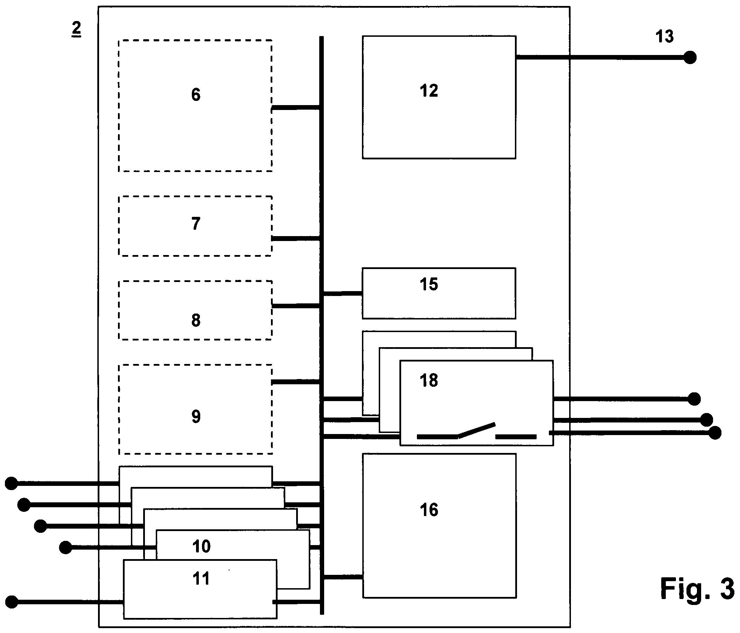

In Fig. 3 ist eine zweite Ausführungsform eines elektronischen Installationsgeräts zur Erfassung und Abspeicherung von Gesundheits-, Fitness-, Wellness- und Vital-Daten dargestellt. Zusätzlich zum elektronischen Installationsgerät 1 gemäß Fig. 2 weist das elektronische Installationsgerät 2 gemäß Fig. 3 mindestens einen Schaltausgang 18 auf.

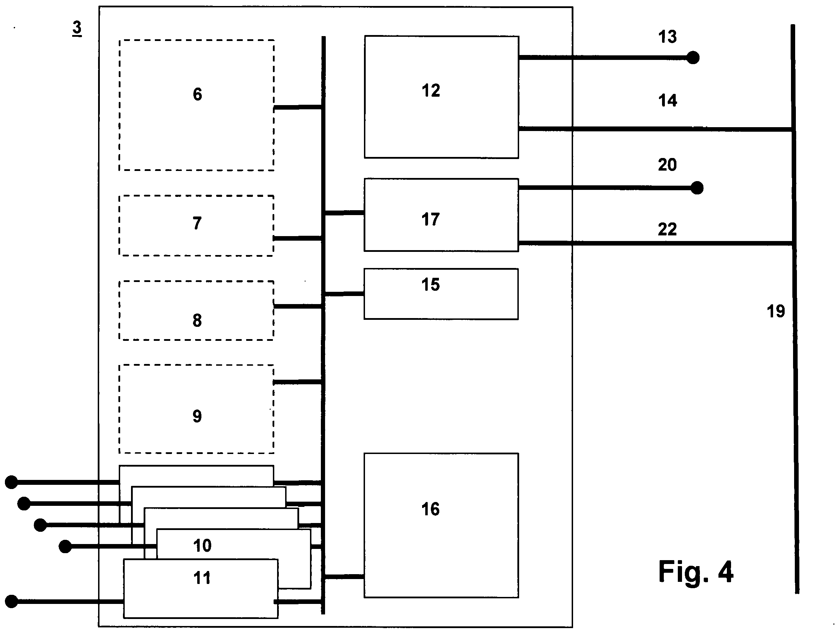

In Fig. 4 ist eine dritte Ausführungsform eines elektronischen Installationsgeräts zur Erfassung und Abspeicherung von Gesundheits-, Fitness-, Wellness- und Vital-Daten dargestellt. Zusätzlich zum elektronischen Installationsgerät 1 gemäß Fig. 2 weist das elektronische Installationsgerät 3 gemäß Fig. 4 eine Busanbindung 17 für den Anschluss an ein Netzwerk/Bussystem 19 der Gebäudesystemtechnik auf. Die Anbindung zwischen Busanbindung 17 und Netzwerk/Bussystem 19 erfolgt wahlweise über eine Drahtlosverbindung 20 oder über einen digitalen Busanschluss 22 für Steuer- und Datenübertragung. Das Netzteil 12 kann wahlweise an die externe Spannungsversorgung 13 oder optional an eine Spannungsversorgung 14 über Busspannung angeschlossen sein, z. B. PoE - "Power over Ethernet" oder KNX.

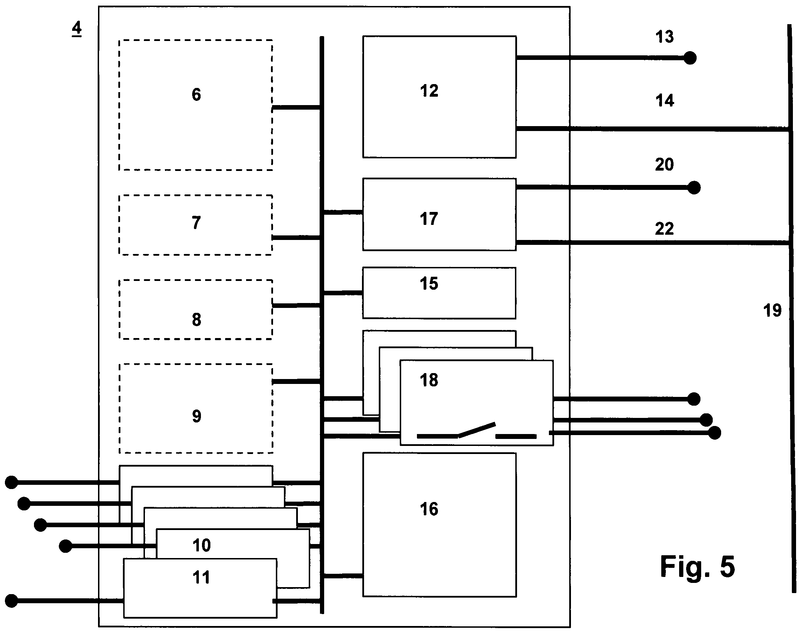

In Fig. 5 ist eine vierte Ausführungsform eines elektronischen Installationsgeräts zur Erfassung und Abspeicherung von Gesundheits-, Fitness-, Wellness- und Vital-Daten dargestellt. Zusätzlich zum elektronischen Installationsgerät 3 gemäß Fig. 4 weist das elektronische Installationsgerät 4 gemäß Fig. 5 mindestens einen Schaltausgang 18 auf.

Sofern die Gesundheits-, Fitness-, Wellness- und Vital-Daten-Geräte 24 - 38 es nicht bereits bieten, können abgeleitete Informationen aus den erfassten / empfangenen Daten der Geräte 24 - 38 im elektronischen Installationsgerät 1 oder 2 oder 3 oder 4 berechnet und dargestellt werden:
- Momentane oder durchschnittliche oder maximale Leistungswerte,
- Gleichmäßigkeit des Kraftverlaufes,
- Links-Rechts-Balance, d. h. die Balance zwischen linkem und rechtem Bein oder zwischen linken und rechtem Arm,
- Gedächtnisleistung und kognitive Leistung anhand der Alphafrequenz.

Sofern die Gesundheits-, Fitness-, Wellness- und Vital-Daten-Geräte 24 - 38 es nicht bereits bieten, können Informationen aus übergreifenden, empfangenen Daten der Gesundheits-, Fitness-, Wellness- und Vital-Daten-Geräte 24 - 38 vom elektronischen Installationsgerät abgeleitet (ausgewertet / verarbeitet) und zur Anzeige gebracht werden:
- Gewichtsreduzierung im Zusammenhang mit den Leistungswerten,
- Muskelmassenaufbau im Zusammenhang mit den Leistungswerten,
- Gewichtsreduzierung in Abhängigkeit von eingenommenen Speisemengen,
- Muskelmassenaufbau in Abhängigkeit von eingenommenen Speisemengen.

Des Weiteren können mittels des elektronischen Installationsgerätes Tagesdosierungen bzw. der Trainingsbedarf des nächsten Tages/Woche vorgeschlagen werden:
- Trainingsbedarf z.B. einer Woche anhand der gewünschten Gewichtsreduzierung basierend auf den persönlichen Informationen der Vergangenheit,
- Trainingsbedarf z.B. des Tages für gewünschten Muskelmassenaufbau.

Ferner können vom elektronischen Installationsgerät Korrelationen zu biologischen Phasen hergestellt werden:
- Gesundheits-, Fitness- und Vitaldaten in Korrelation zum Biorhythmus, Organuhr oder Mondphasen, um die Heilungskraft / Entspannungsfähigkeit / Leistungsfähigkeit des Körpers / Geistes zu bestimmen bzw. vorausschauend abzuleiten.

Eine Personifizierung der Daten sowie Mandantenfähigkeit des elektronischen Installationsgerätes 1 oder 2 oder 3 oder 4 ist ebenfalls möglich - siehe die Personalisierungs-Schnittstelle 11 des elektronischen Installationsgerätes für die Eingabe von Personendaten:
- Name
- Alter / Geburtstag (inkl. Stunde und Minute)
- Geschlecht
Die Eingabe erfolgt am elektronischen Installationsgerät 1 oder 2 oder 3 oder 4 z. B. mittels Fingerprint-Gerät, RF-ID, über das Bedienelement 9 usw.

Über die Personalisierungs-Schnittstelle 11 des elektronischen Installationsgerätes können des Weiteren mögliche Ziele / Veränderungen hinterlegt werden:
- gewünschte Gewichtsreduzierung in (Kilo-) Gramm,
- gewünschter Muskelmassenaufbau in Gramm,
- gewünschte Trainingszeiten in (Stunden-) Minuten pro Tag/Woche/Monat,
- gewünschte Schwellwerte für Blutzuckergehalt, Herzfrequenz, Körpertemperatur.

Über die Personalisierungs-Schnittstelle 11 des elektronischen Installationsgerätes können ferner Zuordnungen und Identifikationen der einzelnen Gesundheits-, Fitness-, Wellness- und Vital-Daten-Geräte 24 - 38, wie insbesondere Blutzuckermessgerät 37, Pulsensor 30, Personenwaage 25 zur automatischen Speicherung der Daten für unterschiedliche Personen hinterlegt werden.

Die Übertragung zwischen den Gesundheits-, Fitness-, Wellness- und Vital-Daten-Geräten 24 - 38 und dem elektronischen Installationsgerät 1 oder 2 oder 3 oder 4 erfolgt, wie bereits erwähnt, kabellos- oder kabelgebunden, wie beispielsweise
- unter Einsatz von Funk (RF), wie WLAN, Bluetooth, ZigBee, 433 MHz und 868 MHz, etc.,
- unter Einsatz von Infrarot (IR),
- unter Einsatz von USB / USB-Stick,
- unter Einsatz von Speicherkarten,
- unter Einsatz von Ethernet (IP).

Das elektronische Installationsgerät 1 oder 2 oder 3 oder 4 kann mit oder ohne Anzeigeeinheit 6 ausgestattet sein. Ohne Anzeigeeinheit 6 dient es der zentralen Erfassung / Speicherung der Daten, der Auswertung der Daten bzw. mit Anzeigeeinheit 6 der Visualisierung der Daten / ausgewerteten Informationen.

Das elektronische Installationsgerät 1 oder 2 oder 3 oder 4 kann mit oder ohne Lautsprecher 7 sowie mit oder ohne Mikrofon 8 ausgestattet sein. Ohne Lautsprecher 7 und/oder Mikrofon 8 dient es der zentralen Erfassung / Speicherung der Daten sowie der Auswertung und Darstellung der Informationen ohne akustische Signalisierung und ggf. Sprachpersonalisierung.

Das elektronische Installationsgerät kann mit einem oder mehreren Schaltausgängen 18 oder ohne Schaltausgänge 18 ausgestattet sein. Mit Schaltausgängen 18 können in Abhängigkeit von mittels der erfassten Daten verbundenen Zuständen und Schwellwerten basierend auf den Gesundheits-, Fitness-, Wellness- und Vital-Daten externe Schaltvorgänge ausgelöst werden.

Das elektronische Installationsgerät kann mit oder ohne Busanbindung 17 ausgestattet sein. Mit Busanbindung 17 besteht die Möglichkeit, in Abhängigkeit von Zuständen und Schwellwerten, basierend auf den Gesundheits-, Fitness-, Wellness- und Vital-Daten, eine direkte Interaktion mit der Gebäudesystemtechnik auszulösen, z.B. eine externe Signalisierung.

Das elektronische Installationsgerät 1 oder 2 oder 3 oder 4 kann mit oder ohne Bedienelement(e) 9 ausgestattet sein. Mit Bedienelementen 9 besteht die Möglichkeit, direkte Eingaben am elektronischen Installationsgerät 1 oder 2 oder 3 oder 4, z.B. Name, Alter, usw. einzugeben, bzw. Schaltvorgänge der optionalen Schaltausgänge 18 oder der Busanbindung 17 auszulösen.

Das elektronische Installationsgerät kann mit oder ohne Anschluss an die Gebäudesystemtechnik ausgestattet sein. Ohne Gebäudesystemtechnikschnittstelle dient es dem oben genannten Zweck ohne Interaktion mit der Gebäudesystemtechnik. Im anderen Fall können Zustände der Gebäudesystemtechnik im Zusammenhang mit Gesundheits-, Fitness-, Wellness- und Vitaldaten gesteuert werden, z.B. Auslösen einer Signalisierung bei Überschreiten eines unter Verwendung der empfangenen Daten berechneten Schwellwertes.

Das elektronische Installationsgerät 1 oder 2 oder 3 oder 4 kann in einen Bedienteil und eine hiervon separierbare, in eine normgerechte UP-Dose montierbare UP-Einheit aufgeteilt werden, wobei diese beiden Einheiten mittels Spannungsschnittstellen und Datenschnittstellen miteinander verbindbar sind. Die Auswahl des Bedienteils und des Abdeckrahmens des elektronischen Installationsgerätes kann nach Wunsch aus unterschiedlichen Installationsgeräte-Programmen mit unterschiedlichem Design und unterschiedlicher Farbgebung erfolgen. Derartige Unterputzprogramme für ein Elektro-Installationssystem sind in unterschiedlicher Formgebung, unterschiedlicher Farbgebung und unter Einsatz unterschiedlicher Materialien (z. B. Kunststoff, Edelstahl) verfügbar. Auf diese Weise wird das elektronische Installationsgerät harmonisch in ein Unterputz-Programm integriert und stellt keinen "Fremdkörper" im Hinblick auf Steckdosen, Schalter, Dimmer, Tastsensoren und Bewegungsmelder dar. Des Weiteren kann das elektronische Installationsgerät in Mehrfach-Kombinationen (z. B. 2fach-Kombination, 3fach-Kombination, 4fach-Kombination) zusammen mit Schaltern, Dimmer usw. eingesetzt werden. Ferner ergeben sich eine Vielzahl unterschiedlicher Kombinationsmöglichkeiten, z. B. im Hinblick auf mögliche Farb-Kombinationen.

### Bezugszeichenliste

- 1: elektronisches Installationsgerät zur Erfassung und Abspeicherung von Gesundheits-, Fitness-, Wellness- und Vital-Daten
- 2: elektronisches Installationsgerät zur Erfassung und Abspeicherung von Gesundheits-, Fitness-, Wellness- und Vital-Daten
- 3: elektronisches Installationsgerät zur Erfassung und Abspeicherung von Gesundheits-, Fitness-, Wellness- und Vital-Daten
- 4: elektronisches Installationsgerät zur Erfassung und Abspeicherung von Gesundheits-, Fitness-, Wellness- und Vital-Daten
- 5: -
- 6: Anzeigeeinheit (optional) zur Visualisierung von Daten (Text, Grafik), Signalisierung (Farbsignalisierung bei Überschreitung von Grenzwerten)
- 7: Lautsprecher (optional)
- 8: Mikrofon (optional)
- 9: Bedienelement (optional) z. B. Taster, Touch Screen
- 10: Erfassungs-Schnittstellen (für Dateneingabe) zu externen Gesundheits-, Fitness-, Wellness- und Vital-Daten-Geräten, z. B. Sensoren / Erfassungsgeräten, kabellos oder kabelgebunden
- 11: Personalisierungs-Schnittstelle
- 12: Netzteil
- 13: externe Spannungsversorgung
- 14: Spannungsversorgung über Busspannung, Z. B. PoE - Power over Ethernet oder KNX
- 15: Steuerlogik/Auswertung/Verarbeitung
- 16: Speicher zum Abspeichern von Daten
- 17: Busanbindung (optional)
- 18: mindestens ein Schaltausgang (optional)
- 19: Netzwerk/Bussystem der Gebäudesystemtechnik
- 20: Drahtlosverbindung
- 21: Abdeckrahmen
- 22: digitaler Busanschluss für Steuer- und Datenübertragung
- 23: -
- 24: Körper-Analyse-Waage
- 25: Personenwaage
- 26: Küchenwaage
- 27: Testgerät für Alkohol oder ähnliche Stoffe
- 28: Lauf-/Trittsensor
- 29: Kraft-/Leistungssensor
- 30: Pulssensor
- 31: Ergometer / Trimmrad
- 32: Wetterstation
- 33: Umweltmesssensoren
- 34: Hautleitsensor
- 35: EEG-Gerät
- 36: Bio-Feedback-Gerät
- 37: Blutzuckermessgerät
- 38: Raumluftsensor

## Patentansprüche

1. Elektronisches Unterputz- Installationsgerät (1, 2, 3, 4) mit einer in eine normgerechte UP-Dose für Unterputzprogramme für ein Elektro-Installationssystem der Installationstechnik für Wohn- und Zweckbau montierbaren UP-Einheit zur Erfassung und Abspeicherung von Gesundheits-, Fitness-, Wellness- und Vital-Daten,
• mit mindestens einer kabellos oder kabelgebunden unter Einsatz von Funk oder Infrarot oder USB / USB-Stick oder Speicherkarten oder Ethernet ausgebildeten Erfassungs-Schnittstelle (10) für die Erfassung von Daten mindestens eines externen Gesundheits-, Fitness-, Wellness- und Vital-Daten-Gerätes (24 - 38),
• mit einem Speicher (16) zum Abspeichern dieser erfassten Daten,
• mit einer Steuerlogik/Auswertung/Verarbeitung (15) zur Auswertung / Verarbeitung dieser erfassten Daten und zur Bereitstellung hierauf basierender Informationen,
• mit einem Netzteil (12),
• mit einer Personalisierungs-Schnittstelle (11) für die Eingabe von Personendaten zur Unterscheidung der Daten unterschiedlicher Geräte-Anwender, und
• mit einer Busanbindung (17) für den Anschluss an ein Netzwerk/Bussystem (19) über eine Drahtlosverbindung (20) oder über einen digitalen Busanschluss (22) für eine Steuer- und Datenübertragung, um basierend auf den Gesundheits-, Fitness-, Wellness- und Vital-Daten, eine direkte Interaktion mit der Gebäudesystemtechnik auszulösen, welche auf einer Gebäudeautomation des Elektro-Installationssystems beruht.

2. Elektronisches Unterputz- Installationsgerät (1, 2, 3, 4) nach Anspruch 1, **dadurch gekennzeichnet, dass** das Netzteil (12) an eine externe Spannungsversorgung (13) angeschlossen ist.

3. Elektronisches Unterputz- Installationsgerät (1, 2, 3, 4) nach Anspruch 1, **dadurch gekennzeichnet, dass** eine Anzeigeeinheit (6) zur Visualisierung von Daten oder zur Signalisierung vorgesehen ist.

4. Elektronisches Unterputz- Installationsgerät (1, 2, 3, 4) nach Anspruch 1, **dadurch gekennzeichnet, dass** mindestens ein Bedienelement (9) vorgesehen ist.

5. Elektronisches Unterputz- Installationsgerät (1, 2, 3, 4) nach Anspruch 1, **dadurch gekennzeichnet, dass** ein Lautsprecher (7) vorgesehen ist.

6. Elektronisches Unterputz- Installationsgerät (1, 2, 3, 4) nach Anspruch 1, **dadurch gekennzeichnet, dass** ein Mikrofon (8) vorgesehen ist.

7. Elektronisches Unterputz- Installationsgerät (1, 2, 3, 4) nach Anspruch 1, **dadurch gekennzeichnet, dass** die Spannungsversorgung über die Busspannung (14) erfolgt.

8. Elektronisches Unterputz- Installationsgerät (1, 2, 3, 4) nach Anspruch 1, **dadurch gekennzeichnet, dass** mindestens ein Schaltausgang (18) vorgesehen ist.

## Claims

1. Electronic flush-mounted installation device (1, 2, 3, 4) having a flush-mounted unit, which is able to be fitted in a standard flush-mount box for flush-mount schemes for an electrical installation system using installation technology for residential and non-residential buildings, for capturing and storing health, fitness, wellness and vital data,
• having at least one capture interface (10), designed in wireless or wired fashion using radio or infrared or USB/USB stick or memory cards or Ethernet, for capturing data of at least one external health, fitness, wellness and vital data device (24 - 38),
• having a memory (16) for storing these captured data,
• having a controller/evaluation/processing unit (15) for evaluating/processing these captured data and for providing information based thereon,
• having a power supply unit (12),
• having a personalization interface (11) for inputting personal data to distinguish the data of different device users, and
• having a bus link (17) for connection to a network/bus system (19) via a wireless connection (20) or via a digital bus connection (62) for a control and data transmission, in order to take the health, fitness, wellness and vital data as a basis for initiating a direct interaction with the building system technology, which is based on building automation of the electrical installation system.

2. Electronic flush-mounted installation device (1, 2, 3, 4) according to Claim 1, **characterized in that** the power supply unit (12) is connected to an external power supply (13).

3. Electronic flush-mounted installation device (1, 2, 3, 4) according to Claim 1, **characterized in that** a display unit (6) is provided for visual display of data or signalling.

4. Electronic flush-mounted installation device (1, 2, 3, 4) according to Claim 1, **characterized in that** at least one operator control element (9) is provided.

5. Electronic flush-mounted installation device (1, 2, 3, 4) according to Claim 1, **characterized in that** a loudspeaker (7) is provided.

6. Electronic flush-mounted installation device (1, 2, 3, 4) according to Claim 1, **characterized in that** a microphone (8) is provided.

7. Electronic flush-mounted installation device (1, 2, 3, 4) according to Claim 1, **characterized in that** the power supply is provided by means of the bus voltage (14).

8. Electronic flush-mounted installation device (1, 2, 3, 4) according to Claim 1, **characterized in that** at least one switching output (18) is provided.

## Revendications

1. Appareil d'installation électronique encastré (1, 2, 3, 4) comprenant une unité encastrée pour l'acquisition et l'enregistrement de données de santé, de forme, de bien-être et de signes vitaux qui peut être montée dans un boîtier encastré normalisé pour des programmes d'encastrement destinés à un système électrique de la technique d'installation pour bâtiments résidentiels et utilitaires,
• comprenant au moins une interface d'acquisition sans fil ou filaire faisant appel à des systèmes radio ou infrarouges ou USB/clé USB ou à carte mémoire ou Ethernet (10) pour l'acquisition de données provenant d'au moins un appareil externe (24 - 38) de données de santé, de forme, de bien-être et de signes vitaux,
• comprenant une mémoire (16) pour l'enregistrement desdites données acquises,
• comprenant une logique de commande / évaluation / traitement (15) pour l'évaluation / le traitement desdites données enregistrées et pour la fourniture d'informations basées sur celles-ci,
• comprenant un bloc d'alimentation (12),
• comprenant une interface de personnalisation (11) pour la saisie de données personnelles afin de distinguer les données de différents utilisateurs de l'appareil, et
• comprenant une connexion par bus (17) pour le raccordement à un réseau / système de bus (19) par l'intermédiaire d'une connexion sans fil (20) ou d'une connexion par bus numérique (22) pour la commande et la transmission de données, afin de déclencher une interaction directe avec le système technique du bâtiment sur la base des données de santé, de forme, de bien-être, et de signes vitaux, qui se fonde sur l'automatisation du bâtiment du système d'installation électrique.

2. Appareil d'installation électronique encastré (1, 2, 3, 4) selon la revendication 1, **caractérisé en ce que** le bloc d'alimentation (12) est raccordé à une alimentation en tension externe (13).

3. Appareil d'installation électronique encastré (1, 2, 3, 4) selon la revendication 1, **caractérisé en ce qu'**il est prévu une unité d'affichage (6) pour la visualisation de données ou pour la signalisation.

4. Appareil d'installation électronique encastré (1, 2, 3, 4) selon la revendication 1, **caractérisé en ce qu'**il est prévu au moins un élément de commande (9).

5. Appareil d'installation électronique encastré (1, 2, 3, 4) selon la revendication 1, **caractérisé en ce qu'**il est prévu un haut-parleur (7).

6. Appareil d'installation électronique encastré (1, 2, 3, 4) selon la revendication 1, **caractérisé en ce qu'**il est prévu un microphone (8).

7. Appareil d'installation électronique encastré (1, 2, 3, 4) selon la revendication 1, **caractérisé en ce que** l'alimentation en tension s'effectue par l'intermédiaire de la tension de bus (14).

8. Appareil d'installation électronique encastré (1, 2, 3, 4) selon la revendication 1, **caractérisé en ce qu'**il est prévu au moins une sortie de commutation (18).
